(19) Europäisches Patentamt
European Patent Office
Office européen des brevets

(11) Veröffentlichungsnummer: **0 008 073**
**A1**

(12) **EUROPÄISCHE PATENTANMELDUNG**

(21) Anmeldenummer: **79102753.5**

(22) Anmeldetag: **01.08.79**

(51) Int. Cl.³: **C 07 D 233/42**, C 07 D 401/04, C 07 D 409/04, A 61 K 31/415, A 61 K 31/44

(30) Priorität: **10.08.78 CH 8526/78**

(43) Veröffentlichungstag der Anmeldung: **20.02.80 Patentblatt 80/4**

(84) Benannte Vertragsstaaten: **AT BE CH DE FR GB IT LU NL SE**

(71) Anmelder: **CIBA-GEIGY AG, Patentabteilung Postfach, CH-4002 Basel (CH)**

(72) Erfinder: **Ferrini, Pier Giorgio, Dr., Im Rehwechsel 22, CH-4102 Binningen (CH)**
Erfinder: **Göschke, Richard, Dr., Felix-Häglistrasse 21, CH-4103 Bottmingen (CH)**

(54) **Thiosubstituierte Diazacycloalkene, Verfahren zu ihrer Herstellung und pharmazeutische Präparate.**

(57) **Neue thiosubstituierte Diazacycloalkene der Formel**

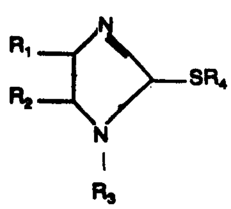

(I),

**worin $R_1$ und $R_2$ unabhängig voneinander gegebenenfalls substituierte Aryl- oder Heteroarylgruppen bedeuten, $R_3$ Wasserstoff oder Niederalkyl darstellt und $R_4$ einen gegebenenfalls substituierten aliphatischen Kohlenwasserstoffrest bedeutet, sowie deren pharmazeutisch verwendbaren Salze mit antiinflammatorischen Eigenschaften. Sie werden beispielsweise hergestellt, indem man Verbindungen der Formeln**

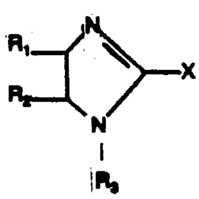

(II) und $Y-R_4$ (III)

worin einer der Reste X und Y gegebenenfalls in Salzform vorliegendes Mercapto und der andere einen gegen veräthertes Mercapto austauschbaren Rest bedeutet, miteinander umsetzt.

EP 0 008 073 A1

0008073

CIBA-GEIGY AG
Basel (Schweiz)

4-11970/+

Thiosubstituierte Diazacycloalkene, Verfahren zu ihrer Herstellung und pharmazeutische Präparate.

Die vorliegende Erfindung betrifft neue thiosubstituierte Diazacycloalkene, insbesondere Verbindungen der Formel

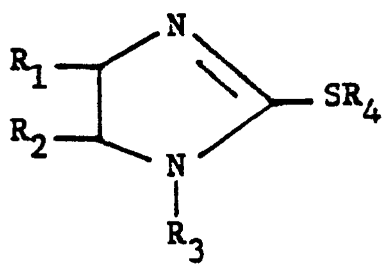 (I) ,

worin $R_1$ und $R_2$ unabhängig voneinander gegebenenfalls substituierte Aryl- oder Heteroarylgruppen bedeuten, $R_3$ Wasserstoff oder Niederalkyl darstellt und $R_4$ einen gegebenenfalls substituierten aliphatischen Kohlenwasserstoffrest bedeutet, und deren Salze, Verfahren zu ihrer Herstellung, Verbindungen der Formel I oder deren pharmazeutisch verwendbaren Salze enthaltende pharmazeutische Präparate, sowie die Verwendung der vorstehend definierten Verbindungen.

Vor- und nachstehend sind unter "niederen" organischen Resten und Verbindungen vorzugsweise solche zu verstehen, die bis und mit 7, vor allem bis und mit 4 Kohlenstoffatome aufweisen.

Arylgruppen sind beispielsweise Phenylgruppen. Heteroarylgruppen sind beispielsweise Thienyl- oder Pyridylgruppen, wie 2- oder 3-Thienyl oder 2-, 3- oder 4-Pyridyl. Als Substituenten derselben kommen beispielsweise Niederalkyl, Niederalkoxy, Halogen, gegebenenfalls substituiertes Amino, Trifluormethyl und/oder Nitro in Betracht. Thienyl-, wie 2-Thienyl-, und Pyridylgruppen $R_1$ und $R_2$ sind vorzugsweise unsubstituiert.

Substituierte Aminogruppen sind mono- oder vorzugsweise disubstituierte Aminogruppen, als deren Substituenten beispielsweise Niederalkyl oder gegebenenfalls durch ein Stickstoff-, Sauerstoff- oder Schwefelatom unterbrochenes Alkylen mit 4 bis 7 Ringgliedern in Frage kommen, wie Niederalkylamino, Diniederalkylamino oder Niederalkylenamino bzw. 3-Aza-, 3-Oxa- oder 3-Thianiederalkylenamino mit jeweils 5 oder 6 Ringgliedern. Als Beispiele seien neben Methylamino und Aethylamino vor allem Dimethylamino, Diäthylamino, Pyrrolidino, Piperidino, Morpholino, Thiomorpholino, Piperazino und N'-Niederalkyl-, z.B. N'-Methylpiperazino genannt.

Aliphatische Kohlenwasserstoffreste sind z.B. gegebenenfalls ungesättigte Niederalkylreste, wie Niederalkyl, Niederalkenyl oder Niederalkinyl.

Als Substituenten von aliphatischen Kohlenwasserstoffresten kommen beispielsweise gegebenenfalls substituierte Aryl-, wie Phenylreste, gegebenenfalls verestertes Carboxy, wie Carboxy oder Niederalkoxycarbonyl, oder in höherer als der α-Stellung gebundenes Hydroxy, Niederalkoxy, Niederalkylthio oder gegebenenfalls substituierte Aryloxy-, wie Phenoxy-, oder Arylthio-, wie Phenylthiogruppen in Betracht.

Aliphatische Kohlenwasserstoffreste sind vorzugsweise gegebenenfalls wie angegeben substituierte, gegebenenfalls ungesättigte Niederalkyl-, Niederalkenyl- oder Niederalkinylreste, z.B. Niederalkyl, Niederalkenyl, gegebenenfalls substituiertes Phenylniederalkyl, Carboxy- bzw. Niederalkoxycarbonylniederalkyl oder in höherer als der α-Stellung durch Halogen, vor allem Fluor, Hydroxy, Niederalkoxy, Niederalkylthio oder durch gegebenenfalls substituiertes Phenoxy oder Phenylthio substituierte Niederalkylreste, wobei mehrere Substituenten, insbesondere mehrere Halogenatome vorhanden sein können.

Gegebenenfalls durch Phenyl substituiertes, gegebenenfalls ungesättigtes Niederalkyl ist beispielsweise Niederalkyl, wie $C_1$-$C_4$-Alkyl, gegebenenfalls wie angegeben substituiertes Phenylniederalkyl, wie 1- oder 2-Phenyl-

$C_1$-$C_4$-alkyl, z.B. Benzyl oder 2-Phenyläthyl, Niederalkenyl, wie $C_2$-$C_4$-Alkenyl, z.B. Vinyl, Allyl oder Methallyl, gegebenenfalls wie angegeben, gegebenenfalls substituiertes Phenylniederalkenyl, wie 2-Phenyl-$C_2$-$C_4$-alkenyl, z.B. Styryl, Niederalkinyl, wie $C_2$-$C_4$-Alkinyl, z.B. Aethinyl oder Propargyl, oder gegebenenfalls wie angegeben substituiertes Phenylniederalkinyl, wie Phenyl-$C_2$-$C_4$-alkinyl, z.B. 2-Phenyläthinyl.

Hydroxyniederalkyl ist beispielsweise Mono- oder Dihydroxyniederalkyl, wie Mono- oder Dihydroxy-$C_2$-$C_4$-alkyl, z.B. 2-Hydroxyäthyl, 2- oder 3-Hydroxypropyl oder 2,3-Dihydroxypropyl.

Halogenniederalkyl ist beispielsweise Mono-, Di- oder Trihalogenniederalkyl, wie 2,2,2-Trichlor- oder 2,2,2-Trifluoräthyl.

Niederalkoxyniederalkyl ist beispielsweise $C_1$-$C_4$-Alkoxyniederalkyl, wie $C_1$-$C_4$-Alkoxy-$C_1$-$C_4$-alkyl, z.B. 2-Methoxyäthyl, 2-Aethoxyäthyl oder 2- oder 3-Methoxypropyl.

Niederalkylthioniederalkyl ist beispielsweise $C_1$-$C_4$-Alkylthio-$C_1$-$C_4$-alkyl, wie 2-Methylthioäthyl.

Phenoxyniederalkyl ist beispielsweise, gegebenenfalls wie angegeben substituiertes Phenoxy-$C_1$-$C_4$-alkyl, wie 2-Phenoxyäthyl, ebenso ist Phenylthioniederalkyl beispielsweise gegebenenfalls wie angegeben substituiertes Phenylthio-$C_1$-$C_4$-alkyl, wie 2-Phenylthioäthyl.

Niederalkyl ist beispielsweise $C_1$-$C_4$-Alkyl, wie Methyl, Aethyl, Propyl, Isopropyl oder n-, iso-, sek- oder tert.-Butyl oder in zweiter Linie eine der isomeren Pentyl-, Hexyl-oder Heptylgruppen.

Niederalkoxy ist beispielsweise $C_1$-$C_4$-Alkoxy, wie Methoxy, Aethoxy, Propoxy, Isopropoxy, n-, sek-, iso- oder tert.-Butoxy oder in zweiter Linie eine der isomeren Pentyl-, Hexyl- oder Heptyloxygruppen.

Niederalkylthio ist beispielsweise $C_1$-$C_4$-Alkylthio, wie Methylthio, Aethylthio, Propylthio, Isopropylthio, Butylthio oder in zweiter Linie Pentylthio, Hexylthio oder Heptylthio.

Niederalkenyl ist beispielsweise $C_2$-$C_4$-Alkenyl, wie Vinyl, Allyl oder Methallyl, während Niederalkinyl z.B. $C_2$-$C_4$-Alkinyl, vor allem Aethinyl oder Propargyl bedeutet.

Halogen ist vorzugsweise Halogen bis und mit Atomnummer 35, d.h. Fluor, Chlor oder Brom.

Salze von Verbindungen der Formel I sind beispielsweise Säureadditionssalze, vor allem pharmazeutisch verwendbare Säureadditionssalze mit starken Säuren, wie Mineralsäure, z.B. Salze mit Halogenwasserstoffsäuren, vor allem Chlor- oder Bromwasserstoffsäure, d.h. Hydrohalogenide, vor allem Hydrochloride und Hydrobromide, oder Schwefelsäuresalze, d.h. Hydrogensulfate und Sulfate.

Die Verbindungen der Formel I sind auf Grund der asymmetrischen C-Atome in 4- und 5-Stellung des Imidazolringes dissymmetrisch und bilden Stereoisomere. Sie treten dementsprechend in mindestens vier stereoisomeren Formen auf, zwei zueinander enantiomeren cis-Formen mit entweder meso- oder (+)- bzw. (-)-erythro-Konfiguration, und zwei zueinander enantiomeren trans-Formen mit entweder D,L- oder (+)-threo- bzw. (-)-threo-Konfiguration, wobei jede cis-Form zu jeder trans-Form diastereomer ist. Bei Verbindungen der Formel I, in denen $R_1$, $R_2$, $R_3$ und/oder $R_4$ asymmetrische Kohlenstoffatome enthalten, sind weitere Isomeriemöglichkeiten in Betracht zu ziehen.

Die Verbindungen der Formel I können dementsprechend in Form eines individuellen Stereoisomeren, z.B. Enantiomeren, oder als Gemische von mindestens zwei Stereoisomeren, z.B. als Diastereomerengemisch, oder Enantiomerengemische, wie Racemate vorliegen.

Die Verbindungen der Formel I zeigen wertvolle pharmakologische Eigenschaften. Insbesondere weisen sie eine ausgeprägte anti-inflammatorische, antithrombotische, immunregulierende und/oder antinociceptive Wirksamkeit auf. So hemmen sie in prophylaktischer Applikation bei zweimaliger Gabe von etwa 100 bis etwa 300 mg/kg p.o. das Kaolinpfoteneoedem der Normalratte und in kurativer Applikation bei viermaliger Gabe von etwa 30 bis 100 mg/kg p.o. das Kaolinpfotenoedem der Ajuvans-Arthritis-Ratte. Ferner hemmen sie das durch Phenyl-p-benzochinon ausgelöste Writhing-Syndrom der Maus und ebenso das durch intravenöse Injektion von 0,2 ml 3%-iger Essigsäure ausgelöste Writhing-Syndrom der Ratte bei prophylaktischer Applikation in Dosen von jeweils etwa 30 bis etwa 100 mg/kg p.o.. Weiterhin sind sie in der experimentellen Lungenembolie des Kaninchens im Dosisbereich von etwa 3 bis etwa 30 mg/kg p.o. wirksam und hemmen in vitro im Konzentrationsbereich von etwa 100 bis etwa 200 mg/l die Prostaglandinsynthese aus Arachidonsäure durch Samenblasenenzyme des Rindes. Vor allem zeigen sie immunregulierende Wirkungen, die sich z.B. im Pertussis-Pfotenoedem der Ratte im Dosisbereich von etwa 3 bis etwa 30 mg/kg p.o. zeigen lässt.

Die Verbindungen der Formel I sind deshalb vorzüglich geeignet als immunregulierende Arzneimittel und zur Behandlung entzündlicher Erkrankungen, vor allem chronischer Entzündungen des rheumatischen Formenkreises, wie der chronischen Arthritis, und/oder als Analgetika.

Die Erfindung betrifft in erster Linie Verbindungen der Formel I, worin $R_1$ und $R_2$ unabhängig voneinander gegebenenfalls substituierte Phenyl-, Thienyl- oder Pyri-

dylgruppen bedeuten, $R_3$ Wasserstoff oder Niederalkyl darstellt und $R_4$ einen gegebenenfalls durch Carboxy bzw. Niederalkoxycarbonyl, z.B. in α-Stellung, gegebenenfalls substituiertes Phenyl oder in einer höheren als der α-Stellung durch Hydroxy, Niederalkoxy, wie Methoxy oder Aethoxy, Niederalkylthio, wie Methylthio, oder eine gegebenenfalls substituierte Phenoxy- oder Phenylthiogruppe einfach oder durch Halogen ein- oder mehrfach substituierten aliphatischen Kohlenwasserstoffrest mit bis und mit 7 Kohlenstoffatomen bedeutet, wobei Phenyl-, Phenoxy-, Phenylthio- und Pyridylgruppen durch Niederalkyl, wie Methyl, Niederalkoxy, wie Methoxy oder Aethoxy, Halogen bis und mit Atomnummer 35, wie Fluor oder Chlor, Trifluormethyl, Nitro und/oder gegebenenfalls Niederalkyl, wie Methyl, oder gegebenenfalls durch Stickstoff, Sauerstoff oder Schwefel unterbrochenes 4- bis 7-gliedriges Alkylen, wie Tetramethylen oder Pentamethylen, als Substituenten enthaltendes Amino substituiert sein können, und deren Salze.

Die Erfindung betrifft vor allem Verbindungen der Formel I, worin $R_1$ und $R_2$ unabhängig voneinander gegebenenfalls substituiertes Phenyl, Thienyl oder Pyridyl bedeuten, $R_3$ Wasserstoff oder in zweiter Linie Niederalkyl, wie Methyl darstellt und $R_4$ gegebenenfalls durch Phenyl oder in einer höheren als der α-Stellung durch Hydroxy, Niederalkoxy, Niederalkylthio, Phenoxy oder Phenylthio einfach oder durch Halogen bis und mit Atomnummer 35 ein- oder mehrfach substituiertes Niederalkyl oder einen gegebenenfalls durch Phenyl substituierten Niederalkenyl- oder Niederalkinylrest bedeutet, wobei Phenyl-, Phenoxy- und Phenylthiogruppen durch Niederalkyl, wie Methyl, Niederalkoxy, wie Methoxy oder Aethoxy, Halogen bis und mit Atomnummer 35, wie Chlor, Nitro, Amino oder N,N-Di-niederalkylamino, wie Dimethylamino, und Pyridylgruppen durch

Niederalkyl, wie Methyl, oder Niederalkoxy, wie Methoxy oder Aethoxy, substituiert sein können, und ihre Salze.

Die Erfindung betrifft vorzugsweise Verbindungen der Formel I, worin $R_1$ und $R_2$ unabhängig voneinander gegebenenfalls durch Niederalkyl mit bis zu 4 C-Atomen, wie Methyl, Niederalkoxy mit bis zu 4 C-Atomen, wie Methoxy, Halogen mit Atomnummer bis und mit 35, wie Chlor, substituiertes Phenyl oder in zweiter Linie unsubstituiertes Pyridyl, wie 2-, 3- oder 4-Pyridyl, bedeuten, $R_3$ Wasserstoff bedeutet, und $R_4$ Niederalkyl mit bis zu 4 C-Atomen, wie Methyl, Aethyl oder Propyl, oder $\omega,\omega,\omega$-Trihalogenniederalkyl mit bis 4 C-Atomen, wie 2,2,2-Trihalogenäthyl, z.B. 2,2,2-Trifluoräthyl, bedeutet, und ihre Salze, vor allem ihre pharmazeutisch verwendbaren Säureadditionssalze.

Die Erfindung betrifft namentlich die in den Beispielen genannten Verbindungen der Formel I.

Die neuen Verbindungen können nach an sich bekannten Verfahren hergestellt werden, beispielsweise indem man Verbindungen der Formeln

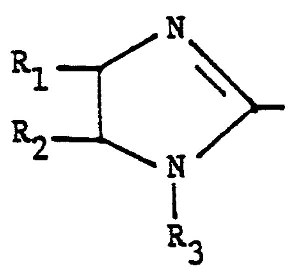 X (II) und Y - $R_4$ (III)

worin einer der Reste X und Y gegebenenfalls in Salzform vorliegendes Mercapto und der andere einen gegen veräthertes Mercapto austauschbaren Rest bedeutet, miteinander oder eine Verbindung der Formel II, worin X die Mercaptogruppe darstellt, mit einem gegebenenfalls wie angegeben substituierten Niederalken umsetzt und gewünschtenfalls

die erhaltene Verbindung in eine andere Verbindung der Formel I umwandelt, ein gegebenenfalls erhaltenes Stereoisomerengemisch in die Komponenten auftrennt und/oder eine erhaltene freie Verbindung in ein Salz oder ein erhaltenes Salz in die freie Verbindung oder in ein anderes Salz überführt.

In Salzform vorliegendes Mercapto ist beispielsweise in Alkalimetallsalz-, z.B. in Natrium- oder Kaliumsalzform, vorliegendes Mercapto.

Gegen veräthertes Mercapto austauschbare Reste X bzw. Y sind beispielsweise Halogenatome, z.B. Chlor, Brom oder Jod, gegen die Gruppe $-SR_4$ austauschbare Reste X sind ferner Sulfonylgruppen, vor allem von organischen Sulfonsäuren abgeleitete Sulfonylgruppen, z.B. Methan-, Aethan-, Benzol-, p-Brombenzol- oder p-Toluolsulfonyl. Gegen $1-R_3-4-R_1-5-R_2$-4-imidazolinyl-(2)-thio austauschbare Reste Y sind ferner andere reaktionsfähig veresterte Hydroxygruppen als Halogen, wie mit Schwefelsäure oder mit einer organischen Sulfonsäure, z.B. mit Methan-, Aethan-, Benzol-, p-Brombenzol- oder p-Toluolsulfonsäure, veresterte Hydroxygruppen.

Die Umsetzung kann in üblicher, insbesondere in der aus der Literatur für analoge Umsetzungen bekannten Weise durchgeführt werden, erforderlichenfalls in Gegenwart eines katalytischen Mittels, bei der Umsetzung von Mercaptoverbindungen der Formel II mit Alkenen oder Phenylalkenen beispielsweise saurer Mittel, wie von Mineralsäuren oder Lewissäuren, z.B. von Bortrifluorid, vorzugsweise in einem Lösungsmittel, bei der Umsetzung von Mercaptoverbindungen der Formel II mit Alkenen oder Phenylalkenen, beispielsweise in Aether, Tetrahydrofuran oder Dioxan oder bei der Umsetzung von Mercaptanen mit Halogeniden beispielsweise in einem Alkohol, z.B. in Methanol,

Aethanol, Aethylenglykol oder Aethylenglykolmonomethyläther, jeweils vorteilhaft unter einer Inertgasatmosphäre, z.B. unter Stickstoff, und erforderlichenfalls bei erhöhter Temperatur, z.B. in der Siedehitze.

Eine bevorzugte Ausführungsform des vorstehenden Verfahrens besteht z.B. darin, dass man ein gegebenenfalls in einer der genannten Salzformen vorliegendes 2-Mercapto-4-imidazolinderivates der Formel II in einem Niederalkanol, z.B. in Methanol oder Aethanol, mit einem Chlor-, Brom- oder Jodwasserstoff- oder Schwefelsäureester der Formel III umsetzt.

Die Ausgangsstoffe sind bekannt oder können in an sich bekannter Weise hergestellt werden.

Verbindungen der Formel II, in denen X Mercapto bedeutet, können z.B. hergestellt werden, indem man eine Verbindung der Formel $R_1$-CH($NH_2$)-CH($NHR_3$)-$R_2$ (IIa) in üblicher Weise mit Schwefelkohlenstoff umsetzt. Verbindungen der Formel IIa, in denen $R_3$ Wasserstoff bedeutet, können z.B. erhalten werden, indem man ein entsprechendes 1,2-Diketon der Formel $R_1$-C(=O)-C(=O)-$R_2$ (IIb) oximiert. und das Dioxim der Formel $R_1$-C(=NOH)-C(=NOH)-$R_2$ (IIc) in üblicher Weise reduziert. Bei Verwendung von Natrium und einem Alkohol als Reduktionsmittel erhält man dabei vorzugweise das meso-Diamin, während durch katalytische Hydrierung überwiegend das D,L-Diamin erhalten wird. Verbindungen der Formel IIa, in denen $R_3$ von Wasserstoff verschieden ist, werden z.B. hergestellt, indem man ein α-Halogenketon, z.B. der Formel $R_2$-CHBr-C(=O)-$R_1$ (IId) zunächst mit dem Amin der Formel $R_3NH_2$ umsetzt, das Reaktionsprodukt, z.B. der Formel $R_2$-CH($NHR_3$)-C(=O)-$R_1$ (IIe) oximiert und das erhaltene Aminooxim, z.B. der Formel $R_2$-CH($NHR_3$)-C(=NOH)-$R_2$ (IIf) in üblicher Weise z.B. wie oben angegeben, reduziert.

Verbindungen der Formel II, in denen X Halogen bedeutet, können z.B. erhalten werden, indem man ein ent-

sprechendes 4-$R_1$-5-$R_1$-Imidazolidin-2-on, erhältlich z.B. aus einem Harnstoff und einem 1-$R_1$-2-$R_2$-1,2-Dibromäthan oder aus einem Diamin der Formel IIa durch Umsetzung mit einem Kohlensäurediester oder -esterchlorid, in üblicher Weise, z.B. mit Phosphortribromid, Phosphorpentachlorid oder dergleichen, in 2-Stellung halogeniert.

Verbindungen der Formel II, worin X Sulfonyl bedeutet, können beispielsweise hergestellt werden, indem man eine Verbindung der Formel

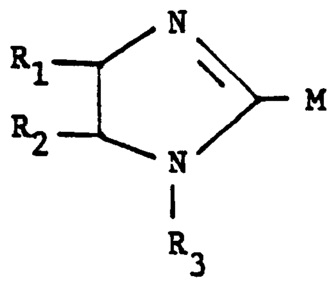 (IIg)

worin M ein Metallradikal, wie ein Alkalimetallatom, z.B. Lithium, oder ein Halogenerdalkalimetallradikal, z.B. der Formel -MgBr, bedeutet, in üblicher Weise mit einem entsprechenden Sulfonylhalogenid, z.B. der Formel X-Cl (IIh) umsetzt. Die dafür als Ausgangsstoffe zu verwendenden Verbindungen der Formel IIg werden vorteilhaft durch übliche Umsetzung einer entsprechenden 2-unsubstituierten 4-$R_1$-5-$R_2$-4-Imidazolinverbindung mit einer Metallkohlenwasserstoffverbindung, z.B. mit Butyllithium oder Butylmagnesiumbromid, <u>in situ</u> hergestellt und ohne Isolierung umgesetzt. Verbindungen der Formel II, in denen X eine von Sulfonyl $R_4$-$SO_2$- verschiedene Sulfonylgruppe $R_4'$-$SO_2$- z.B. gegebenenfalls substituiertes Benzolsulfonyl, wie p-Toluolsulfonyl, bedeutet, können ferner hergestellt werden, indem man in einer Verbindung der Formel

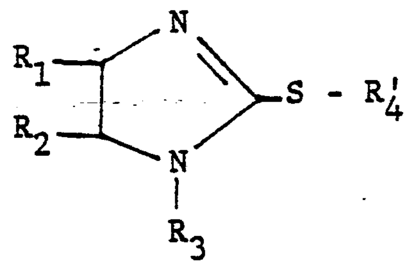

(IIi)

die $R_4'$-S-Gruppe in üblicher Weise, z.B. durch Umsetzung mit einer Hydroperoxyverbindung, z.B. mit m-Chlorperbenzoesäure oder Wasserstoffperoxid in Essigsäure, zu $-SO_2R_4'$ oxydiert. Die dafür als Ausgangsstoffe zu verwendenden Verbindungen der Formel (IIi) können beispielsweise hergestellt werden, indem man eine Verbindung der Formel

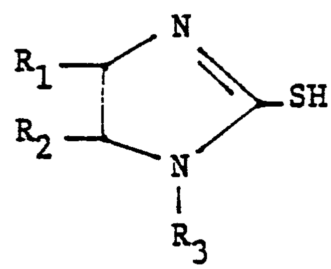

(II')

oder vorzugsweise ein Alkalimetallsalz, wie das Natriumsalz, davon mit einer Verbindung der Formel $R_4'Hal$, worin Hal Halogen, wie Chlor, Brom oder Jod bedeutet, umsetzt und gewünschtenfalls erhaltene Verbindungen der Formel II, worin $R_3$ Wasserstoff bedeutet, durch Umsetzung mit einem Niederalkylhalogenid der Formel $R_3$-Hal in 1-Stellung substituiert.

Die neuen Verbindungen können ferner hergestellt werden, indem man eine Verbindung der Formel

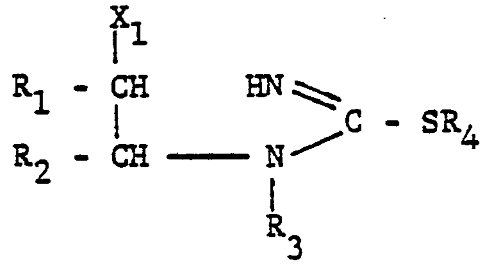

(IV) ,

worin $X_1$ einen gegen Imino austauschbaren Rest bedeutet, oder ein Salz davon cyclisiert und gewünschtenfalls die erhaltene Verbindung in eine andere Verbindung der Formel I umwandelt, ein gegebenenfalls erhaltenes Stereoisomerengemisch in die Komponenten auftrennt und/oder eine erhaltene freie Verbindung in ein Salz oder ein erhaltenes Salz in die freie Verbindung oder in ein anderes Salz überführt.

Gegen Imino austauschbare Reste $X_1$ sind beispielsweise gegebenenfalls veresterte Hydroxygruppen oder Aminogruppen. Veresterte Hydroxygruppen sind beispielsweise reaktionsfähig veresterte Hydroxygruppen, wie Halogen, z.B. Chlor oder Brom, oder Sulfonyloxygruppen, z.B. Methan-, Benzol- oder p-Toluolsulfonyloxy.

Salze von Verbindungen der Formel IV sind beispielsweise deren Säureadditionssalze mit Mineralsäuren, wie deren Hydrohalogenide oder Sulfate.

Die Cyclisierung erfolgt in üblicher Weise, beispielsweise in Gegenwart eines Kondensationsmittels, wie einer anorganischen Base, wie eines Alkalimetallhydroxides, -carbonates oder -hydrogencarbonates, z.B. von Natrium- oder Kaliumhydroxid, Kaliumcarbonat oder Natriumhydrogencarbonat, oder einer tertiären organischen Stickstoffbase, z.B. von Triäthylamin oder Pyridin, vorzugsweise in einem unter den Reaktionsbedingungen inerten Lösungsmittels, erforderlichenfalls bei erhöhter oder erniedrigter Temperatur, z.B. bei etwa 0° bis etwa 100°C.

Die Ausgangsstoffe der Formel IV werden vorteilhaft <u>in situ</u> hergestellt. So kann man beispielsweise ein Diamin der Formel $R_1$-CH($NH_2$)-CH($NR_3$)-$R_2$ (IIa) oder ein entsprechendes 1-$R_1$-2-$R_2$-äthylenoxid mit einem $R_4$-Isothioharnstoff oder dem entsprechenden Isothiuroniumsalz, z.B. einem entsprechenden Hydrohalogenid, zu einer Verbindung der Formel III umsetzen, worin $X_1$ Amino ist. Das Diamin der Formel IIa kann z.B. erhalten werden, indem man ein Bromketon der Formel $R_1$-C(=O)-CHBr-$R_2$ (IId)

mit einem $R_3$-Amin umsetzt, das Reaktionsprodukt der Formel $R_1-C(=O)-CH(NHR_3)-R_2$ (IIe) bzw. ein Diketon der Formel $R_1-C(=O)-C(=O)-R_2$ (IIb) oximiert und das so erhältliche Oxim der Formel $R_1-C(=NOH)-CH(NHR_3)-R_2$ (IIf) bzw. $R_1-C(=NOH)-C(=NOH)-R_2$ in üblicher Weise reduziert. Analog kann man ein Dihalogenid der Formel $R_1-CHX_1-CHX_1-R_2$ (IVa), worin $X_1$ Halogen, z.B. Brom darstellt mit einem $R_4$-Isothioharnstoff oder dem entsprechenden Isothiuroniumsalz umsetzen. Dihalogenide, vor allem Dichloride der Formel IIIa sind beispielsweise zugänglich, indem ein entsprechendes Olefin der Formel $R_1-CH=CH-R_2$ (IVb) in üblicher Weise an der Doppelbindung halogeniert, z.B. chloriert.

Zur Herstellung von Verbindungen der Formel IV, in denen $X_1$ Hydroxy ist, geht man vorteilhaft von einem entsprechenden α-Halogenketon, z.B. der Formel $R_1-C(=O)-CHBr-R_2$ (IId) aus, setzt dieses mit einem S-$R_4$-Thioharnstoff oder dem entsprechenden Isothiuroniumsalz um und reduziert in dem Kondensationsprodukt der Formel

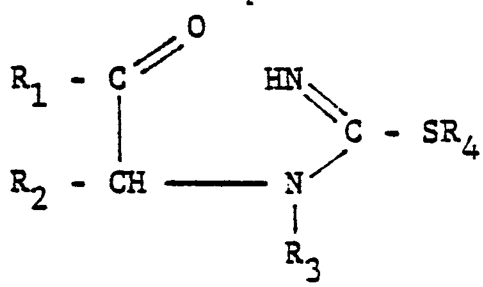

(IVc) .

die Carbonylgruppe in üblicher Weise, z.B. mit Natriumborhydrid oder Natriumcyanoborhydrid, zu Hydroxymethylen.

Die Verbindungen der Formel I können ferner hergestellt werden, indem man in einer Verbindung der Formel

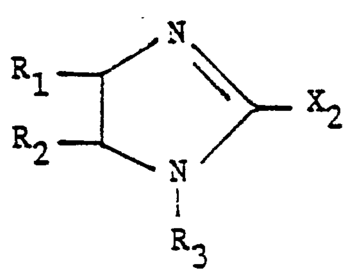

(V) ,

worin $X_2$ einen in einen Rest $R_4S$- überführbaren Rest bedeutet, $X_2$ in den gewünschten Rest $R_4S$- überführt und gewünschtenfalls die erhaltene Verbindung in eine andere Verbindung der Formel I umwandelt, ein gegebenenfalls erhaltenes Stereoisomerengemisch in die Komponenten auftrennt und/oder eine erhaltene freie Verbindung in ein Salz oder ein erhaltenes Salz in die freie Verbindung oder in ein anderes Salz überführt.

In Reste der Formel $R_4S$- überführbare Gruppen $X_2$ sind beispielsweise solche der Formel $R_4''$-$S(O)_n$-, worin n 0, 1 oder 2 bedeutet und $R_4''$ einen in $R_4$ überführbaren Rest oder, wenn n 1 oder 2 darstellt, einen Rest $R_4$ bedeutet. In Reste $R_4$ überführbare Gruppen sind beispielsweise im aliphatischen Teil durch gegebenenfalls verestertes oder anhydridisiertes Carboxy, Epoxy, funktionell abgewandeltes Hydroxy oder Oxo und/oder Ammonium- oder Sulfoniumgruppen substituierte Reste $R_4$. Verestertes Carboxy ist beispielsweise mit einem aliphatischen, araliphatischen oder aromatischen Alkohol verestertes Carboxy, wie Niederalkoxycarbonyl oder gegebenenfalls substituiertes Phenoxycarbonyl. Anhydridisiertes Carboxy ist beispielsweise mit einer Halogenwasserstoffsäure anhydridisiertes Carboxy, wie Halogencarbonyl. Funktionell abgewandeltes Hydroxy ist beispielsweise verestertes Hydroxy, wie mit einer Carbonsäure oder organischen Sulfonsäure verestertes Hydroxy, wie Niederalkanoyloxy, z.B. Acetoxy, oder gegebenenfalls substituiertes Benzoyloxy, Alkan- oder gegebenenfalls substituierten Benzolsulfonyloxy, z.B. Methan-, Aethan-, Benzol-, p-Toluol- oder Mesitylensulfonyloxy. Funktionell abgewandeltes Oxo ist beispielsweise gegebenenfalls in β-Stellung durch 2-Sulfonyl, z.B. p-Toluolsulfonyl oder Mesitylensulfonyl, substituiertes Hydrazono. Ammoniumgruppen sind beispielsweise di- oder trisubstituierte Ammoniumgruppen, z.B. Di- oder Triniederalkylammoniumgruppen oder Diniederalkylaminoxidgruppierungen. Sulfoniumgruppen sind beispielsweise disubstituierte Sulfoniumgruppen, wie Diniederalkylsulfoniumgruppen oder Niederalkan- bzw. gegebenenfalls substituiertes Benzolsulfinyl bzw- sulfonyl, wie Methan-, Aethan-, Benzol-, p-Toluol- oder Mesitylensulfinyl oder entsprechendes Sulfonyl.

Die Ueberführung von Gruppen $X_2$ in solche der Formel $R_4S$- erfolgt in üblicher Weise, z.B. durch Reduktions-, Solvolyse-, Substitutions- und/oder Eliminierungsmethoden.

Durch Reduktion kann man beispielsweise in einer Gruppe $X_2$ der Formel $R_4''-S(O)_n-$, worin n 0, 1 oder 2 bedeutet und $R_4''$ einen durch gegebenenfalls verestertes oder anhydridisiertes Carboxy, gegebenenfalls im aliphatischen Teil substituiertes Hydrazono, Oxo und/oder verestertes Hydroxy substituierten oder, wenn n 1 oder 2 bedeutet, unsubstituierten Rest $R_4$ bedeutet, Sulfinyl bzw. Sulfonyl in Thio, gegebenenfalls verestertes oder anhydridisiertes Carboxy in gegebenenfalls C-substituiertes Hydroxymethyl und/oder Oxo, Epoxy bzw. mit einer Carbonsäure verestertes Hydroxy in Hydroxy überführen und/oder Oxo, gegebenenfalls substituiertes Hydrazono bzw. mit einer organischen Sulfonsäure verestertes Hydroxy durch Wasserstoff ersetzen.

Die Reduktion kann durch Umsetzung mit einem üblichen Reduktionsmittel erfolgen, beispielsweise mit einem Leichtmetallhydrid oder Dileichtmetallhydrid, wie einem Boran, z.B. Diboran oder dem Boran-Tetrahydrofurankomplex, einem Alkalimetallborhydrid, z.B. mit Natriumborhydrid, Lithiumborhydrid oder Natriumcyanoborhydrid, einem Aluminiumhydrid, z.B. Triisobutylaluminiumhydrid, oder einem Alkalimetallaluminiumhydrid, z.B. mit Lithiumaluminiumhydrid. So kann man mit Lithiumaluminiumhydrid beispielsweise Oxo, Epoxy oder mit einer Carbonsäure verestertes Hydroxy zu Hydroxy, gegebenenfalls verestertes oder anhydridisiertes Carboxy zu Hydroxymethyl und Sulfinyl bzw. Sulfonyl zu Thio reduzieren und endständiges Sulfonyloxy durch Wasserstoff ersetzen. Dabei arbeitet man in üblicher Weise, beispielsweise in einem gegenüber den Reaktionsteilnehmern inerten Lösungsmittel, erforderlichenfalls bei erhöhter oder erniedrigter Temperatur, z.B. bei etwa 0 bis 100°C. Die Reduktion von Oxo zu Hydroxy, anhydridisiertem Carboxy zu Hydroxymethyl und von Sulfinyl zu Thio kann ferner durch Umsetzung mit Natriumborhydrid oder Natriumcyanoborhydrid, vorteilhaft in einem Alkohol, wie einem Niederalkanol, z.B. in Aethanol, oder einem Carbonsäureamid, wie N-Methylpyrrolidon, N,N-Dimethylformamid oder N-Methylacetamid, oder einem, vorzugsweise wasserhaltigen Gemisch derselben, erfolgen. Zur Reduktion von Oxo zu Hydroxy, Carboxy zu Hydroxymethyl und Sulfinyl bzw. Sulfonyl zu Thio kann man jedoch beispielsweise auch Diboran und zum Ersatz einer endständigen Sulfonyloxygruppe durch Wasserstoff auch Triisobutylaluminiumhydrid verwenden. Die Reduktion von Oxo zu Hydroxy und von gegebenenfalls verestertem oder anhydridisiertem Carboxy zu C-substituiertem Hydroxymethyl kann durch Umsetzung mit einer aliphatischen, araliphatischen oder heteroaliphatischen Alkalimetall- oder Erdalkalimetallverbindung, wie einer entsprechenden Lithium- oder Halogenmagnesiumverbindung erfolgen, vorteilhat in einem ätherartigen Lösungsmittel, z.B. in Diäthyläther, Tetrahydrofuran oder Dioxan, erforderlichenfalls unter Kühlen oder Erwärmen, z.B. bei etwa 0° bis 100°C, durchgeführt werden. Oxo wird dabei zu sekundärem Hydroxy und gegebenenfalls verestertes oder anhydridisertes Carboxy zu C-substituiertem Hydroxymethyl reduziert. Die vorstehenden Reduktionen werden vorteilhaft unter Inertgas, wie Stickstoff, erforderlichenfalls im geschlossenen Gefäss durchgeführt.

Der reduktive Ersatz von Oxo durch Wasserstoff kann beispielsweise durch nascierenden Wasserstoff erfolgen, der vorteilhaft durch Einwirkung von Protonendonatoren auf unedle Metalle, z.B. von Protonensäuren, wie Salzsäure oder Essigsäure, auf Eisen, Zink oder Aluminium, oder von Wasser oder Alkoholen, wie Aethanol, oder deren Gemische auf amalgamiertes Aluminium oder Zink von Wasser auf Natriumamalgam oder von Alkoholen, wie Methanol, auf Natrium, in situ erzeugt wird. Der reduktive Ersatz von gegebenenfalls substituiertem Hydrazono durch Wasserstoff erfolgt vorzugsweise durch Disproportionierung, beispielsweise durch Erhitzen mit einer Metallbase, wie einem Alkalimetallhydroxid oder- alkoholat, z.B. mit Kaliumhydroxid oder Natriummethanolat, vorteilhaft in einem hochsiedenden Lösungsmittel, wie einem hochsiedenden Alkohol, z.B. von Aethylenglykol, Aethylenglykolmonomethyläther, Diäthylenglykol oder Diäthylenglykolmonomethyläther, auf etwa 100 bis 250°C, vorteilhaft unter Inertgas.

Durch Solvolyse, wie Hydrolyse oder Alkoholyse in Gegenwart eines sauren oder basischen Mittels kann man beispielsweise mit einer Carbonsäure verestertes Hydroxy in Hydroxy überführen. Als saure Mittel verwendet man vorzugsweise Protonensäuren, wie Mineralsäuren, z.B. Salz- oder Schwefelsäure. Geeignete basische Mittel sind beispielsweise Metallbasen, bei der Hydrolyse vorzugsweise Alkalimetallhydroxide, z.B. Natriumhydroxid oder Kaliumhydroxid, gegebenenfalls in wässrigem Methanol oder Aethanol, oder bei der Alkoholyse vorzugsweise Alkalimetallalkoholate, vor allem solche von niedrigsiedenden Alkoholen, wie Natriummethanolat.

Durch Substitution kann man beispielsweise gegebenenfalls in Säureanhydridform vorliegendes Carboxy in Trihalogenmethyl überführen bzw. Oxo durch zwei Halogenatome ersetzen. Dazu setzt man mit üblichen Halogenierungsmitteln, z.B. mit Phosphorpentachlorid oder mit Schwefeltetrafluorid, um. Zur Ueberführung von Carboxy in Trifluormethyl ist es dabei ökonomischer die Carboxygruppe zunächst durch Umsetzung mit einem Sekundäraminoschwefeltrifluorid, z.B. Diäthylaminoschwefeltrifluorid in Fluorcarbonyl zu überführen und dieses dann mit Schwefeltetrafluorid zu perfluorieren. Weiterhin kann man eine Carboxygruppe durch Wasserstoff ersetzen, vorzugsweise durch Decarboxylierung, vorzugsweise unter Erhitzen auf etwa 100 bis 250°C, erforderlichenfalls in Gegenwart von Kupferverbindungen und/oder in einem hochsiedenden Lösungsmittel, wie Diphenyläther, Dimethylformamid oder Diäthylenglykolmono- oder -dimethyläther.

Durch Eliminierung können z.B. β-Sulfonylhydrazonogruppen sowie Ammonium- bzw. Sulfoniumgruppen zusammen mit einem vicinalen Wasserstoffatom unter Bildung einer Doppelbindung abgespalten werden. Die Eliminierung erfolgt in üblicher Weise durch Basebehandlung und/oder Erhitzen auf etwa 100 bis 250°C. Als Basen verwendet man ausgehend von β-Sulfonylhydrazonogruppen beispielsweise starke Metallbasen, wie Lithiumkohlenwasserstoffverbindungen, wie Butyllithium, und ausgehend von Di- oder Triniederalkylammonium- bzw. Diniederalkylsulfoniumgruppen schwach nukleophile Basen, wie Alkalimetallhydroxide, z.B. Natriumhydroxid.

Die Ausgangsstoffe der Formel V, in denen $X_2$ einen Rest der Formel $R_4''$-S- bedeutet, können beispielsweise hergestellt werden, indem man eine Verbindung der Formel

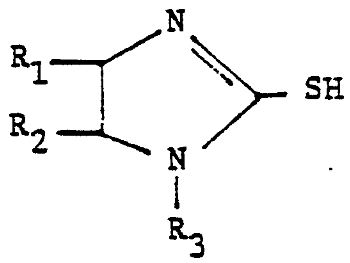

(II')

oder vorzugsweise ein Alkalimetallsalz, wie das Natriumsalz davon mit einer Verbindung der Formel $R_4''$-Hal, worin Hal Halogen, wie Chlor, Brom oder Jod bedeutet, umsetzt und gewünschtenfalls eine erhaltene Verbindung der Formel IV, worin $R_3$ Wasserstoff darstellt, durch Umsetzung mit einem Niederalkylhalogenid der Formel $R_3$-Hal in 1-Stellung substituiert.

Ausgangsstoffe der Formel V, in denen $X_2$ einen Rest $R_4''$-S- und $R_4''$ einen einen Sulfonium- oder Ammoniumgruppe aufweisenden Rest bedeutet, können vorteilhaft aus entsprechenden, z.B. wie vorstehend beschrieben erhältlichen, Halogenverbindungen erhalten werden, indem man diese entweder mit einem di- oder trisubstituierten Amin, vorteilhaft in Gegenwart eines basischen Kondensationsmittels, z.B. von Triäthylamin, umsetzt gegebenenfalls eine trisubstituierte Aminogruppe anschliessend quaterniesiert oder, z.B. mit m-Chlorperbenzoesäure, in eine Diniederalkylaminoxidgruppierung überführt, oder mit einem Niederalkylmercaptid bzw. gegebenenfalls substituierten Phenylmercaptid oder Diniederalkylsulfid umsetzt und gewünschtenfalls Niederalkylthio niederalkyliert oder Niederalkyl- oder gegebenenfalls substituiertes Phenylthio, z.B. mit m-Chlorperbenzoesäure, zu entsprechendem Sulfinyl oder Sulfonyl oxidiert. Analog kann man auch im Rest $R_4''$ eine gegebenenfalls substituierte Hydrazonogruppe aufweisende Verbindung der Formel IV

vorteilhaft durch Umsetzung einer entsprechenden Oxoverbindung der Formel IV mit gegebenenfalls substituiertem Hydrazin herstellen.

Erfindungsgemäss erhältliche Verbindungen der Formel I können in üblicher Weise in andere Verbindungen der Formel I überführt werden.

So kann man beispielsweise in die Reste $R_1$ und $R_2$ sowie die aromatische Gruppen als Bestandteil von $R_4$ Nitrogruppen in üblicher Weise, wie mit nascierendem Wasserstoff, z.B. mit Eisen/Salzsäure, zu Amino reduzieren.

Aminogruppen können ferner in üblicher Weise substituiert, z.B. durch Umsetzung mit einem Alkylierungsmittel, wie einem der genannten in Gegenwart eines basischen Kondensationsmittels alkyliert werden.

Ferner können Aminogruppen durch Behandeln mit Salpetersäure und einer Halogenwasserstoffsäure, wie Chlor-, Brom- oder Jodwasserstoffsäure, erforderlichenfalls in Gegenwart von Kupfer oder einer Kupfer-I-verbindung, durch Halogen ersetzt werden. In analoger Weise kann man Amino durch Umsetzung mit salpetriger Säure und Erwärmen des so erhältlichen Diazoniumsalzes mit Wasser oder einem Niederalkanol in Hydroxy bzw. Niederalkoxy überführen. Ferner kann Hydroxy in üblicher Weise, z.B. durch Umsetzung mit einem Niederalkylierungsmittel, z.B. einem Niederalkylhalogenid oder Diniederalkylsulfat, erforderlichenfalls in Gegenwart von ——————— Kaliumcarbonat, vorteilhaft in einem Keton, wie Aceton, oder einem höhersiedenden Alkohol, wie Amylalkohol, zu Niederalkoxy substituiert werden. Umgekehrt kann man Niederalkoxy durch übliche Säurebehandlung, beispielsweise Einwirkung von Jodwasserstoffsäure oder Bromwasserstoff in Essigsäure, in Hydroxy überführen.

Ferner kann man in Verbindungen der Formel I, worin $R_3$ Wasserstoff darstellt, in 1-Stellung Niederalkyl einführen, beispielsweise durch übliche Umsetzung mit einem Niederalkylierungsmittel, wie einem reaktionsfähigen Ester eines Niederalkanols, beispielsweise einen Niederalkylhalogenid, z.B. Niederalkylchlorid, -bromid oder -jodid, vorteilhaft in Gegenwart eines basischen Kondensationsmittels, wie eines Alkalimetallhydroxides, -alkoholates oder -amides, z.B. von Natrium- oder Kaliumhydroxid, einem Natriumniederalkanolat, wie Natriummethanolat, Natriumamid oder Lithiumdiisopropylamid. Die N-Niederalkylierung kann aber auch durch Umsetzung mit einem Oxoniederalkan, wie einem Niederalkanal oder Diniederalkylketon in Gegenwart eines Reduktionsmittels erfolgen. Als Reduktionsmittel eignen sich insbesondere Dileichtmetallhydride, wie Natriumborhydrid oder Natriumcyanoborhydrid, sowie Formaldehyd bzw. Ameisensäure und ihre Salze.

Ferner kann man in Verbindungen der Formel I Reste $R_4$ in andere Reste $R_4$ überführen.

Beispielsweise kann man im aliphatischen Teil enthaltene Dreifach- zu Doppelbindungen und/oder Doppelbindungen zu Einfachbindungen reduzieren, beispielsweise durch Einwirkung von Wasserstoff in Gegenwart eines Hydrierungskatalysators, wie eines Uebergangsmetalles, z.B. von Raney-Nickel, Palladium oder Platin auf Kohle, Platinoxid, Triphenylphosphin-Rhodiumchlorid oder zur Reduktion von Dreifach- zu Doppelbindungen von Platin auf Silicagel, vorteilhaft in einem inerten Lösungsmittel, z.B. in Aethanol, Aceton oder Essigsäure, erforderlichenfalls unter erhöhtem Druck und/oder erhöhter Temperatur. Ungesättigte Reste $R_4$ können aber auch durch Addition von Wasser, Halogenwasserstoff oder Halogen in substituierte gesättigte Reste $R_4$ überführt werden. So kann man durch übliche Addition von Wasser an Doppel- bzw. Dreifachbindungen Hydroxy bzw. Oxo aufweisende, durch übliche Addition von Halogenwasserstoff an Doppel- bzw. Dreifachbindungen Halogen bzw. geminales Dihalogen aufweisende und durch übliche Addition von Halogen an Doppel- bzw. Dreifachbindungen vicinales Dihalogen bzw. vicinal-geminales Tetrahalogen aufweisende, substituierte, im aliphatischen Teil gesättigte Reste $R_4$ erhalten.

Ferner kann man in Oxo aufweisenden Resten $R_4$ Oxo reduktiv durch Wasserstoff ersetzen, beispielsweise durch Einwirkung von nascierendem Wasserstoff, beispielsweise erzeugt durch Behandlung unedler Metalle mit Protonendonatoren, z.B. von Zink mit Salz- oder Essigsäure, von amalgamiertem Aluminium oder Natriumamalgam mit Wasser oder von Natrium mit einem Alkohol, wie Methanol. Der reduktive Ersatz von Oxo durch Wasserstoff kann auch erfolgen durch Umsetzung mit Hydrazin in Gegenwart einer Metallbase, z.B. von Natrium- oder Kaliumhydroxid oder eines Alkalimetallalkoholates, wie von Natriummethanolat in einem hochsiedenden Alkohol, wie Aethylenglykol, Diäthylenglykol oder Diäthylenglykolmonomethyläther, vorzugsweise bei erhöhter Temperatur, z.B. bei etwa 150 bis 250°C. Oxo kann ferner in üblicher Weise zu Hydroxy reduziert werden, beispielsweise durch Umsetzung mit einem Leichtmetallhydrid, wie Boran-Tetrahydrofuran oder Diboran, oder einem Dileichtmetallhydrid, wie Natriumborhydrid, Natriumcyanoborhydrid oder Lithiumaluminiumhydrid, oder durch Umsetzung mit einem sekundären Alkohol, wie Isopropanol oder Cyclohexanol, in Gegenwart eines Aluminiumalkoholates. Die Reduktion von

Oxo zu Hydroxy kann aber auch unter gleichzeitiger Einführung eines Kohlenwasserstoffrestes am Carbonylkohlenstoffatom erfolgen, indem man mit einer Aliphatyl- oder gegebenenfalls substituierten Phenylaliphatyl-, Heteroaryl- oder Phenylmetallverbindung, z.B. gegebenenfalls substituiertem Phenyllithium oder Phenylmagnesiumhalogenid oder vor allem einem Niederalkyllithium oder Niederalkylmagnesiumhalogenid, umsetzt. In analoger Weise kann man durch Aldoladdition eines Ketons oder Aldehydes an eine aldehydische Oxogruppe unter Reduktion derselben zu Hydroxy einen in β-Stellung Oxo aufweisenden Rest einführen. Oxo kann ferner durch Behandlung mit einem geeigneten Halogenierungsmittel, wie Phosphorpentachlorid oder einem Diniederalkylaminoschwefeltrifluorid, welches durch Umsetzung eines Diniederalkyl-trimethylsilyl-amins mit Schwefeltetrafluorid in Diäthyläther leicht zugänglich ist, durch geminales Dihalogen ersetzt werden. Oxo kann ferner durch Umsetzung mit einem Phosphorylid, z.B. mit einem Triphenyl-niederalkylidenphosphoran, durch einen zweiwertigen Kohlenwasserstoffrest, wie Niederalkyliden, ersetzt werden. In analoger Weise kann man durch Umsetzung mit einem Triphenyl-niederalkoxymethylen-phosphoran und Hydrolyse der intermediär gebildeten Enoläthers Oxo durch Formyl und Wasserstoff ersetzen.

Weiterhin kann man in Hydroxy aufweisenden Resten $R_4$ die Hydroxygruppe reduktiv durch Wasserstoff ersetzen, beispielsweise wie oben für den reduktiven Ersatz von Oxo durch Einwirkung von Wasserstoff angegeben. Hydroxy kann man ferner ~~durch~~ Umsetzung mit einer oxidierenden Schwermetallverbindung, z.B. mit Silberacetat oder Wismutoxid, mit Dimethylsulfoxid in Gegenwart von Trifluormethansulfonsäureanhydrid oder N-Chlorsuccinimid, oder mit einem Keton, wie Aceton oder

Cyclohexanon, in Gegenwart eines Aluminiumalkoholates, wie Aluminiumisopropylat, zu Oxo oxydieren. Ferner kann man Hydroxy durch Umsetzung mit üblichen Halogenierungsmitteln, wie Thionylchlorid oder einem Diniederalkylamino-schwefeltrifluorid, in Halogen überführen. Ein Hydroxy aufweisender Rest kann ferner durch übliche, z.B. säurekatalysierte, Wasserabspaltung in den entsprechenden im aliphatischen Teil ungesättigten Rest $R_4$ überführt werden.

In Resten $R_4$ kann ferner Halogen durch übliche, beispielsweise durch eine schwach nukleophile Metallbase, wie Natronlauge, katalysierte, Hydrolyse in Hydroxy überführt werden. Analog kann man geminale Dihalogenverbindungen zu den entsprechenden Oxoverbindungen hydrolysieren. Halogen kann ferner durch Umsetzung mit geeigneten Leichtmetall- oder Dileichtmetallhydriden, z.B. mit einem Triniederalkylaluminiumhydrid oder mit Lithiumaluminiumhydrid in Gegenwart von Aluminiumtrichlorid, reduktiv durch Wasserstoff oder durch Umsetzung mit einer metallorganischen Verbindung, wie einem Niederalkyllithium oder Niederalkylmagnesiumhalogenid, reduktiv durch einen Kohlenwasserstoffrest ersetzt werden. Halogen aufweisende Reste $R_4$ können aber auch durch Halogenwasserstoffabspaltung in entsprechende ungesättigte Reste $R_4$ überführt werden.

Erfindungsgemäss erhältliche Stereoisomerengemische können in üblicher Weise in die Komponenten aufgetrennt werden.

So kann man Diasteromerengemische auf Grund der Unterschiede der physikalischen Eigenschaften der Komponenten durch übliche physikalische Trennverfahren, wie Kristallisations-, Chromatographie-, Destillations- oder Phasenverteilungsverfahren in die Komponenten auftrennen.

Enantiomerengemische, wie Racemate, können durch Kristallisation aus optisch aktiven Lösungsmitteln, Chromatographie an optisch aktiven Feststoffen, Einwirkung von Mikroorganismen oder Umsetzung mit einer optisch aktiven Hilfsverbindung in Diastereomerengemische, z.B. mit einer optisch aktiven Säure in Gemische diastereomerer Säureadditionssalze überführt werden und durch Trennung derselben in die Diastereomeren, aus denen die Enantiomeren in der jeweils üblichen Weise freigesetzt werden können, in die Enantiomeren gespalten werden. Für diesen Zweck übliche optisch aktive Säuren sind z.B. D- oder L-Weinsäure, Di-o-Toluylweinsäure, Aepfelsäure, Mandelsäure, Camphersulfonsäuren oder Chinasäure.

Ferner können erhaltene freie Verbindungen in an sich bekannter Weise in Säureadditionssalze überführt werden, z.B. durch Umsetzen einer Lösung der freien Verbindung in einem geeigneten Lösungsmittel oder Lösungsmittelgemisch mit einer der vorgenannten Säuren oder mit einer Lösung davon, oder mit einem geeigneten Anionenaustauscher.

Erhaltene Säureadditionssalze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder mit einem geeigneten Anionenaustauscher.

Erhaltene Säureadditionssalze können in an sich bekannter Weise in andere Säureadditionssalze überführt werden, z.B. durch Behandlung eines Salzes einer anorganischen Säure mit einem geeigneten Metallsalz, wie einem Natrium-, Barium- oder Silbersalz, einer Säure in einem geeigneten Lösungsmittel, in welchem ein sich bildendes anorganisches Salz unlöslich ist und damit aus dem Reaktionsgemisch ausscheidet, in andere Säureadditionssalze übergeführt werden.

Die Verbindungen, einschliesslich ihrer Salze können auch in Form der Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckmässig gegebenenfalls auch die entsprechenden Salze bzw. freien Verbindungen zu verstehen.

Die Erfindung betrifft auch diejenigen Ausführungsformen des Verfahrens, bei denen man von einer auf beliebiger Verfahrensstufe als Zwischenprodukte erhältlichen Verbindung ausgeht und die fehlenden Verfahrensschritte ausführt, oder wobei ein Ausgangsstoff unter den Reaktionsbedingungen gebildet oder in Form eines Derivats davon, gegebenenfalls eines Salzes, verwendet wird.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe sowie Verfahren zu ihrer Herstellung bilden ebenfalls einen Gegenstand der vorliegenden Erfindung.

Bei den erfindungsgemässen pharmazeutischen Präparaten, welche Verbindungen der Formel I oder pharmazeutisch verwendbare Salze davon enthalten, handelt es sich um solche zur enteralen wie oralen oder rektalen, und parenteralen Verabreichung sowie zur topischen Anwendung an Warmblüter(n), welche den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise ab.

Im Normalfall ist für einen etwa 75 kg schweren Warmblüter bei oraler Applikation eine ungefähre Tagesdosis von etwa 30-300 mg, vorteilhaft in mehreren gleichen Teildosen verteilt, zu veranschlagen.

Die neuen pharmazeutischen Präparate enthalten z.B. von etwa 10% bis etwa 80%, vorzugsweise von etwa 20% bis etwa 60% des Wirkstoffs. Erfindungsgemässe pharmazeutische Präparate zur enteralen bzw. parenteralen Verabreichung sind z.B. solche in Dosiseinheitsformen wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzü-

gen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaftresistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglycolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglycole oder höhere Alkanole. Ferner können auch Gelatine-Rektalkapseln verwendet werden, die eine Kombination des Wirkstoffs mit einer Grundmasse enthalten; als Grundmassenstoffe kommen z.B.

flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls auch Stabilisatoren enthalten.

Als topisch anwendbare pharmazeutische Präparate kommen in erster Linie Creme, Salben, Pasten, Schäume, Tinkturen und Lösungen, in Frage, die von etwa 0,5 bis etwa 20% des Wirkstoffs enthalten.

Creme sind Oel-in-Wasser-Emulsionen, die mehr als 50% Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Aethylenoxidaddukte davon, wie Polyglycerinfettsäureester oder Polyoxyäthylensorbitan-fettsäureester (Tweens), ferner Polyoxyäthylenfettalkoholäther oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart

von Fettalkoholen, z.B. Cetylalkohol oder Stearylalkohol, verwendet. Zusätze zur Wasserphase sind u.a. Mittel, welche die Austrocknung der Creme vermindern, z.B. Polyalkohole, wie Glycerin, Sorbit, Propylenglykol und/oder Polyäthylenglykole, ferner Konservierungsmittel, Riechstoffe etc.

Salben sind Wasser-in-Oel-Emulsionen, die bis zu 70%, vorzugsweise jedoch von etwa 20% bis etwa 50% Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs enthalten. Emulgatoren sind entsprechende lipophile Substanzen, wie Sorbitan-fettsäureester (Spans), z.B. Sorbitanoleat und/ oder Sorbitanisostearat. Zusätze zur Wasserphase sind u.a. Feuchhaltungsmittel, wie Polyalkohole, z.B. Glycerin, Propylenglykol, Sorbit und/oder Polyäthylenglykol, sowie Konservierungsmittel, Riechstoffe, etc.

Fettsalben sind wasserfrei und enthalten als Grundlage insbesondere Kohlenwasserstoffe, z.B. Paraffin, Vaseline und/oder flüssige Paraffine, ferner natürliche oder partialsynthetische Fette, z.B. Kokosfettsäuretriglyceride, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernde Fettalkohole, Emulgatoren und/oder Zusätze.

Pasten sind Creme und Salben mit sekretabsorbierenden Puderbestandteilen, wie Metalloxiden, z.B. Titanoxid oder Zinkoxid, ferner Talk und/oder Aluminiumsilikate,

welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorofluorniederalkane, z.B. Dichlordifluormethan und Dichlortetrafluoräthan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyäthylen-sorbitan-fettsäureester (Tweens), und solchen mit vorwiegend liphophilen Eigenschaften, wie Sorbitanfettsäureester (Spans). Dazu kommen die üblichen Zusätze, wie Konservierungsmittel, etc.

Tinkturen und Lösungen weisen meistens eine wässrig-äthanolische Grundlage auf, der u.a. Polyalkohole, z.B. Glycerin, Glycole, und/oder Polyäthylenglykol, als Feuchthaltemittel zur Herabsetzung der Verdunstung, und rückfettende Substanzen, wie Fettsäureester mit niedrigen Polyäthylenglycolen, d.h. im wässrigen Gemisch lösliche, lipophile Substanzen als Ersatz für die der Haut mit dem Aethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel von der Emulgierung in einer der beiden Phasen gelöst; bei Verarbeitung als Suspension wird er nach der Emulgierung mit einem Teil der Grundlage vermischt und dann dem Rest der Formulierung begegeben.

Die vorliegende Erfindung betrifft ebenfalls die Verwendung der Verbindungen der Formel I und der Salze von solchen Verbindungen mit salzbildenden Eigenschaften, vorzugsweise zur Behandlung von Entzündungen, in erster Linie von entzündlichen chronischen Erkrankungen des rheumatischen Formenkreises, besonders der chronischen Arthritis.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1

Man löst 1,8 g trans-4,5-Diphenyl-imidazolidin-2-thion in 20 ml Methanol, gibt eine Lösung von 0,3 g Natriumhydroxid in 2 ml Wasser sowie 1,2 g Methyljodid hinzu, lässt 4 Stunden bei Raumtemperatur rühren und über Nacht bei 0° stehen. Die Reaktionsmischung wird zur Trockne eingedampft, der Rückstand mit einem Gemisch von 40 ml Wasser und 40 ml Essigsäureäthylester geschüttelt, die organische Phase abgetrennt und die wässrige Phase mit Essigsäureäthylester nachgewaschen. Die organischen Phasen werden über Natriumsulfat getrocknet und zur Trockne eingedampft. Der kristalline Rückstand wird nacheinander aus Methanol und einem Gemisch von Diäthyläther und Petroläther umkristallisiert. Man erhält das trans-2-Methylthio-4,5-diphenyl-imidazolin vom F. 135-137°.

Das Ausgangsmaterial kann z.B. folgendermassen hergestellt werden:

11,5 g D,L-1,2-Diphenyläthylendiamin (F. 70-71°), erhältlich durch Reduktion von Benzildioxim mit Natrium in Aethanol, werden in 230 ml Schwefelkohlenstoff gelöst. Die Lösung wird 3 Stunden gerührt und zur Trockne eingedampft. Der Rückstand wird in einem Gemisch von 40 ml Aethanol und 40 ml Wasser aufgenommen, mit 0,6 ml konzentrierter Salzsäure versetzt und 48 Stunden zum Rückfluss erhitzt. Dann wird zur Trockne eingedampft und aus einem Gemisch von Aethanol und Aceton umkristallisiert. Man erhält das trans-4,5-Diphenyl-imidazolidin-2-thion vom F. 223-225°.

Beispiel 2

10,5 g cis-4,5-Diphenyl-imidazolidin-2-thion werden in 120 ml Dimethylformamid gelöst. Man fügt 1,9 g Natriumhydroxid in 12 ml Wasser hinzu, verdünnt mit 200 ml Dimethylformamid und gibt 7 g Methyljodid hinzu. Man

lässt 4 Stunden bei Raumtemperatur rühren, lässt über Nacht bei 0° stehen, dampft zur Trockne ein und schüttelt mit einem Gemisch von 200 ml Wasser und 200 ml Essigsäureäthylester durch. Die zurückbleibenden Kristalle werden abfiltriert und mit Methanol ausgekocht. Durch Einengen der methanolischen Lösung erhält man das cis-2-Methylthio-4,5-diphenyl-imidazolin-hydrojodid vom F. 232-245° (Zers.). Dieses kann durch Auflösen in 60 ml Methanol. Versetzen mit 30 ml 2n-Natronlauge, Einengen und Abfiltrieren in das cis-2-Methylthio-4,5-diphenyl-imidazolin vom F. 132-135° überführt werden.

Die oben erhaltene Essigsäureäthylesterlösung wird von der wässrigen Phase abgetrennt, über Natriumsulfat getrocknet und eingedampft. Das zurückbleibende Oel wird mit Methanol aufgekocht, vom Unlöslichen abfiltriert und eingedampft. Man erhält das cis-1-Methyl-2-methylthio-4,5-diphenyl-imidazolin vom F. 148°(Zers.).

Das als Ausgangsmaterial verwendete cis-4,5-Diphenyl-imidazolidin-2-thion kann in analoger Weise wie in Beispiel 1 beschrieben ausgehend von meso-1,2-Diphenyläthylendiamin hergestellt werden. Es schmilzt bei 252°. Das meso-Diphenyläthylendiamin kann durch Hydrierung von Benzildioxim in einem Gemisch aus Aethanol und n-Natronlauge in Gegenwart von Palladium (5%-ig auf Kohle) bei Normaldruck und Raumtemperatur erhalten werden.

Beispiel 3

In analoger Weise wie in Beispiel 1 und 2 beschrieben erhält man ferner:

cis-2-Aethylthio-4,5-diphenyl-imidazolin, Fp. 94-6°,

trans-2-Aethylthio-4,5-diphenyl-imidazolin, Fp. 130-2°,
cis-4,5-bis-(p-Methoxyphenyl)-2-methylthioimidazolin,
Fp. 163-64°,
trans-4,5-bis-(p-Methoxyphenyl)-2-methylthioimidazolin,
cis-2-Aethylthio-4,5-bis-(p-chlorphenyl)-imidazolin-hydrochlorid, Fp. 226-7°,
trans-2-Aethylthio-4,5-bis-(p-chlorphenyl)-imidazolin,
cis-4,5-Bis-(p-chlorphenyl)-2-methylthio-imidazolin-hydrochlorid, Fp. 224° Zers.,
trans-4,5-Bis-(p-chlorphenyl)-2-methylthioimidazolin,
cis-2-Aethylthio-4,5-bis-(p-methoxyphenyl)-imidazolin,
Fp. 92-3°,
trans-2-Aethylthio-4,5-bis-(p-methoxyphenyl)-imidazolin
und
trans-2-Propylthio-4,5-diphenyl-imidazolin, Fp. 133-35°

<u>Beispiel 4</u>

2,5 g D,L-trans-4,5-Diphenyl-imidazolidin-2-thion werden in 25 ml Methanol gelöst mit 1,7 g Methyljodid versetzt und 15 Stunden zum Rückfluss erhitzt. Dann gibt man weitere 0,4 g Methyljodid hinzu und erhitzt nochmals 5 Stunden zum Rückfluss. Dann wird zur Trockne eingedampft und mit konzentrierter, wässriger Ammoniaklösung aufgeschlämmt. Das ausgefallene bzw. ungelöst zurückbleibende <u>D,L-trans-2-Methylthio-4,5-diphenyl-imidazolin</u> wird abfiltriert und aus Aethanol umkristallisiert. Es schmilzt bei 135-137°.

Das Ausgangsmaterial kann in analoger Weise wie in Beispiel 1 beschrieben hergestellt werden.

Das D,L-1,2-Diphenyläthylendiamin wird jedoch vorteilhafter durch Acetylieren von 2,4,5-Triphenyl-4,5-dihydro-imidazol mittels Acetanhydrid und Natriumacetat und anschliessende Behandlung mit Salzsäure hergestellt. Das danach primär erhältliche D,L-1,2-Diphenyläthylendiamin-bis-hydrochlorid schmilzt bei 247-249°, das durch Umsetzung der Base mit Schwefelkohlenstoff erhältliche D,L-trans-4,5-Diphenyl-imidazolidin-2-thion bei 221-223°.

In analoger Weise kann man ausgehend von 2,4,5-Triphenyl-4,5-dihydro-imidazol über D,L-1,2-Diphenyläthylendiamin, Aufspaltung desselben in literaturbekannter Weise in D-1,2-Diphenyläthylendiamin und L-1,2-Diphenyläthylendiamin, Umsetzung derselben zunächst mit Schwefelkohlenstoff und anschliessend mit Methyljodid das

D-trans-2-Methylthio-4,5-diphenyl-imidazolin sowie das

L-trans-2-Methylthio-4,5-diphenyl-imidazolin herstellen.

Beispiel 5

17,5 g cis-4,5-Diphenyl-imidazolidin-2-thion werden in 200 ml Dimethylformamid gelöst und zunächst mit einer Lösung von 3,2 g Natriumhydroxid in 20 ml Wasser und anschliessend mit 11,6 g Methyljodid versetzt. Man lässt 6,5 Stunden bei Raumtemperatur rühren, dampft zur Trockne ein, verrührt mit 350 ml Wasser, saugt nach einigem Stehenlassen ab, löst das noch feuchte Mischgut in 350 ml Aethanol und fügt 50 ml 2n-Natronlauge und 170 ml Wasser hinzu. Der kristalline Niederschlag wird abgesaugt, über Kieselgel mit Chloroform als Elutionsmittel filtriert und aus Isopropanol/Petroläther umkristallisiert. Man erhält das cis-2-Methylthio-4,5-diphenyl-imidazolin vom Smp. 132-134°.

Beispiel 6

9,4 g cis-4,5-Bis-(p-methoxyphenyl)-imidazolin-2-thion werden in 150 ml Dimethylformamid gelöst und zunächst mit 15 ml 2n-Natronlauge und anschliessend mit 1,86 ml Methyljodid in 10 ml Dimethylformamid versetzt. Man lässt 12 Stunden bei Raumtemperatur rühren, fügt nochmals 10 ml 2n-Natronlauge und 1,24 ml Methyljodid, in 10 ml Dimethylformamid gelöst, hinzu und lässt weitere 12 Stunden rühren. Dann wird in Wasser eingegossen, abfiltriert, in 200 ml Chloroform gelöst, über Natriumsulfat getrocknet und bis zur Trübung eingeengt. Nach einiger Zeit erfolgt Kristallisation. Das Kristallisat wird abgesaugt, über Kieselgel mit Chloroform als Elutionsmittel filtriert und aus Isopropanol/Petroläther umkristallisiert. Man erhält das cis-2-Methylthio-4,5-bis-(p-methoxyphenyl)-imidazolin vom Smp. 132-134°.

Das Ausgangsmaterial kann z.B. folgendermassen hergestellt werden:

67,7 g meso-1,2-Bis-(p-methoxyphenyl)-äthylendiamin, erhältlich durch Hydrierung von p-Anisoindioxin in Gegenwart von Palladium auf Kohle, Smp. 151-152,5°, werden in 1400 ml Aethanol gelöst, mit 27 ml Schwefelkohlenstoff versetzt und 16 Stunden unter Rühren zum Rückfluss erhitzt. Die ausgefallenen Kristalle werden abgenutscht, mit 1200 ml Aethanol aufgeschlämmt und bis zum Aufhören der Schwefelwasserstoffentwicklung (etwa 30 Stunden) zum Rückfluss erhitzt. Man erhält das cis-4,5-Bis-(p-methoxyphenyl)-imidazolidin-2-thion vom Smp. 226-227°.

Beispiel 7

12 g cis-4,5-Bis-(p-methoxyphenyl)-imidazolidin-2-thion werden zu einer Lösung von 0,88 g Natrium in 13 ml Methanol gegeben. Dann wird mit 200 ml Dimethylformamid verdünnt, über Nacht stehengelassen, mit 4 ml Aethyljodid versetzt, 5 Stunden bei Raumtemperatur gerührt und in 300 ml Wasser gegossen. Die ausgefallenen Kristalle werden abgesaugt und in Methylenchlorid gelöst. Die Lösung wird über Natriumsulfat getrocknet, eingedampft und der Rückstand in Aceton gelöst. Man sauert mit methanolischer Salzsäure (4,5 N) schwach an und filtriert das ausgefallene cis-2-Aethyl-thio-4,5-bis-(p-methoxyphenyl)-imidazolin-hydrochlorid ab. Zur Freisetzung der Base wird dieses mit 2n-Natronlauge behandelt und die Rohbase anschliessend aus Isopropanol/Petroläther umkristallisiert. Man erhält das cis-2-Aethylthio-4,5-bis-(p-methoxyphenyl)-imidazolin vom Smp. 92-93°.

Beispiel 8

10,3 g cis-4,5-Diphenyl-imidazolidin-2-thion werden in einem Gemisch aus 25 ml Methanol und 3,8 ml Aethyljodid suspendiert und 1 Stunde unter Rühren zum Rückfluss erhitzt. Dann gibt man weitere 75 ml Methanol hinzu und lässt 16 Stunden am Rückfluss sieden. Die klare Reaktionslösung wird zur Trockne eingedampft und der Rückstand in einem Gemisch aus 150 ml Wasser und 250 ml Essigsäureäthylester aufgenommen. Zur Freisetzung der Base wird mit konzentrierter wasseriger Ammoniaklösung alkalisch gemacht, gut durchgeschüttelt, die organische Phase abgetrennt, über Natriumsulfat getrocknet und eingedampft. Man erhält nach Umkristallisieren aus Isopropanol/Petroläther das cis-2-Aethylthio-4,5-diphenyl-imidazolin vom Smp. 95-96°.

In analoger Weise kann man durch Umsetzung mit Propyljodid das cis-4,5-Diphenyl-2-propylthio-imidazolin.

Beispiel 9

5 g cis-4,5-Bis-(p-chlorphenyl)-imidazolidin-2-thion werden in 100 ml Dimethylformamid gelöst, zunächst mit 0,7 g Natriumhydroxid in 4,4 ml Wasser und dann mit 1,3 g Methyljodid versetzt und über Nacht stehengelassen. Dann wird in Wasser gegossen und nochmals mit Essigsäureäthylester ausgezogen. Die Auszüge werden vereinigt, über Natriumsulfat getrocknet und eingedampft. Das zurückbleibende Oel wird über eine Kieselgelsäule mit Chloroform als Laufmittel chromatographisch gereinigt. Man erhält das cis-2-Methylthio-4,5-bis-(p-chlorphenyl)-imidazolin, das zur charakterisierung durch Behandeln mit äthanolischer Salzsäure in das cis-2-Methylthio-4,5-bis-(p-chlorphenyl)-imidazolin-Hydrochlorid vom Smp. 224° überführt werden kann

Das Ausgangsmaterial kann z.B. folgendermassen hergestellt werden:

22,8 g meso-1,2-Bis-(p-chlorphenyl)-äthylendiamin, auf bekannte Weise zugänglich aus p-Chlorbenzaldehyd durch Umsetzung mit Ammoniumacetat und anschliessende Behandlung mit verdünnter Schwefelsäure, Smp. 136-138° werden in 350 ml Aethanol gelöst, mit 8,7 ml Schwefelkohlenstoff versetzt und 12 Stunden zum Rückfluss erhitzt. Die Lösung wird eingeengt und das cis-4,5-Bis-(p-chlorphenyl)-imidazolidin-2-thion nach erfolgter Kristallisation abgesaugt. Es schmilzt, aus Methanol umkristallisiert, bei 197-208°.

Beispiel 10

2,2 g cis-4,5-Bis-(p-chlorphenyl)-imidazolidin-2-thion werden mit 20 ml Methanol und 0,9 g Aethyljodid versetzt und 22 Stunden zum Rückfluss erhitzt. Man lässt abkühlen, nutscht die farblosen Kristalle ab, nimmt in 100 ml Wasser und 100 ml Essigsäureäthylester auf, gibt bis zur alkalische Reaktion konzentrierte wässerige Ammoniaklösung hinzu, trennt die organische Phase ab, trocknet über Natriumsulfat, dampft zur Trockne ein und chromatographiert an Kieselgel mit Methylenchlorid als Laufmittel. Nach dem Eindampfen erhält man das cis-2-Aethylthio-4,5-bis-(p-chlorphenyl)-imidazol als Oel, das zur Charakterisierung in Isopropanol als Lösungsmittel durch Behandlung mit einer Lösung von Chlorwasserstoff in Essigsäureäthylester in das cis-2-Aethylthio-4,5-bis-(p-chlorphenyl)-imidazolin-hydrochlorid vom Smp. 226-227° überführt werden kann.

Beispiel 11

2,5 g DL-trans-4,5-diphenyl-imidazolidin-2-thion werden mit 25 ml Methanol und 1,85 g Aethyljodid versetzt und 15 Stunden zum Rückfluss erhitzt. Dann fügt man weitere 0,4 g Aethyljodid hinzu und lässt nochmals 5 Stunden am Rückfluss sieden. Dann wird zur Trockne eingedampft. Man erhält das DL-trans-2-Aethylthio-4,5-diphenyl-imidazolin-hydrojodid. Zur Ueberführung in die Base wird dieses in 100 ml Essigester und 100 ml Wasser suspendiert, die Suspension mit konzentrierter wässeriger Ammoniaklösung alkalisch gemacht, die organische Phase abgetrennt, über Natriumsulfat getrocknet und eingedampft. Man erhält das DL-trans-2-Aethylthio-4,5-diphenyl-imidazolin vom Smp. 130-132°.

In analoger Weise kann man ausgehend von D- bzw. L-trans-4,5-Diphenyl-imidazolidin-2-thion das

D-trans-2-Aethylthio-4,5-diphenyl-imidazolin

bzw. das

L-trans-2-Aethylthio-4,5-diphenyl-imidazolin herstellen.

Beispiel 12

4,3 g cis-2-(2-Acetoxyäthyl)-4,5-bis-(p-methoxyphenyl)-imidazolin werden mit 100 ml Methanol und 25 ml 2n-Natronlauge 4 Stunden zum Rückfluss erhitzt. Man filtriert heiss vom Unlöslichen ab, dampft zur Trockne ein und kristallisiert aus Isopropanol/ Petroläther um. Man erhält das cis-2-(2-Hydroxyäthyl)-4,5-bis-(p-methoxyphenyl)- imidazolin als Oel.

Das Ausgangsmaterial kann z.B. folgendermassen hergestellt werden:

8 g cis-4,5-Bis-(p-methoxyphenyl)-imidazolidin-2-thion werden in 50 ml Methanol mit 3,5 g 2-Acetoxyäthylchlorid 22 Stunden zum Rückfluss erhitzt. Man dampft zur Trockne ein, nimmt in 100 ml Essigester und 25 ml Wasser auf, fügt bis zur alkalischer Reaktion konzentrierte wässerige Ammoniaklösung hinzu, trennt die organische Phase ab, trocknet über Natriumsulfat und dampft zur Trockne ein. Man erhält das cis-2-(2-Acetoxyäthyl)-4,5-bis-(p-methoxyphenyl)-imidazolin, das ohne weitere Reinigung eingesetzt werden kann.

Beispiel 13

7,5 g cis-1-Methyl-4-phenyl-5-(3-pyridyl)-imidazolidin-2-thion werden in 100 ml Methanol in 5,5 g

Aethyljodid 22 Stunden zum Rückfluss erhitzt. Man dampft zur Trockne ein, nimmt in 100 ml Essigester und 25 ml Wasser auf, fügt bis zur alkalischer Reaktion konzentrierte wässerige Ammoniaklösung hinzu, trennt die organische Phase ab, trocknet über Natriumsulfat und dampft zur Trockne ein. Man erhält das cis-2-Aethylthio-1-methyl-4-phenyl-5-(3-pyridyl)-imidazolin.

Das Ausgangsmaterial lässt sich wie folgt erhalten:

13,5 g Benzyl-pyridyl(3)-keton werden zusammen mit 50 ml Pyridin und einer Lösung von 10 g Hydroxylaminhydrochlorid in 20 ml Pyridin 6 Stunden bei 100° gerührt. Das Reaktionsgemisch wird auf Eis/Wasser gegossen und 15 Minuten weitergerührt. Die ausgefallenen Kristalle werden abgenutscht, mit Wasser gewaschen und am Hochvakuum getrocknet. Man erhält das Benzyl-pyridyl(3)-keton-oxim vom F. 122-126°.

Zu einer bei -10° gerührten Lösung von 6,4 g Benzyl-pyridyl(3)-keton-oxim in 15 ml Pyridin wird innert 5 Minuten die Lösung von 5,8 g p-Toluolsulfochlorid in 15 ml Pyridin zugetropft. Das Reaktionsgemisch wird 24 Stunden im Eisschrank aufbewahrt und dann auf Eis/Wasser gegossen. Nach längerem Rühren und Verreiben erstarrt das ausgefallene Oel zu Kristallen. Diese werden abgenutscht, mit Wasser gewaschen und am Hochvakuum getrocknet. Man erhält 11,6 g eines Rohproduktes vom F. 87-92°, welches nach Dünnschichtchromatographie jedoch noch Edukt enthält. Es wird direkt in der nächsten Stufe eingesetzt.

8,7 g Rohprodukt (Benzyl-pyridyl(3)-keton-oxim-p-toluolsulfoester) werden in 70 ml absolutem Aethanol suspendiert und bei 0° unter Rühren die Lösung von 2,8 g Kalium-tert.-butylat in 25 ml absolutem Aethanol zugetropft. Das Reaktionsgemisch wird 2 Stunden bei 0° gerührt. Die Suspension wird abgenutscht und das Filtrat sofort in der nächsten Stufe eingesetzt.

Die das α-Amino-benzyl-(3-pyridyl)-keton enthaltende Lösung wird mit 2,4 g Methylisothiocyanat versetzt und 3 Stunden bei 0-5° gerührt. Dann werden portionsweise 2,3 g Natriumborhydrid hinzugegeben und bei 0-10° 4 Stunden gerührt. Man dekantiert vom überschüssigem Natriumborhydrid ab, fügt 2 ml Aceton hinzu und erhitzt 18 Stunden zum Rückfluss. Nach dem Abkühlen lässt sich aus dem Reaktionsgemisch 2,1 g rohes 1-Methyl-4-p-Phenyl-5-pyridyl(3)-imidazolidin-2-thion abnutschen. Das Filtrat enthält weitere Mengen des Produktes.

## Beispiel 14

Tabletten enthaltend 25 mg Wirkstoff, z.B. cis-2-Methylthio-4,5-diphenyl-imidazolin, können folgendermassen hergestellt werden:

| Bestandteile (für 1000 Tabletten) | |
|---|---|
| Wirkstoff | 25,0 g |
| Lactose | 100,7 g |
| Weizenstärke | 7,5 g |
| Polyäthylenglykol 6000 | 5,0 g |
| Talkum | 5,0 g |
| Magnesiumstearat | 1,8 g |
| entmineralisiertes Wasser | q.s. |

Herstellung

Sämtliche festen Ingredienzien werden zunächst durch ein Sieb von 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyäthylenglykols in 100 ml Wasser hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

In analoger Weise können auch Tabletten, enthaltend jeweils 25 mg einer anderen der in den Beispielen 1 bis 3 genannten Verbindungen der Formel I hergestellt werden.

Beispiel 15

In analoger Weise wie in Beispiel 14 beschrieben kann man auch Tabletten enthaltend 25 mg einer gemäss einem der Beispiele 4-13 erhältlichen Verbindung herstellen.

Patentansprüche

(für alle benannten Länder ausser Oesterreich).

1. Verbindungen der Formel

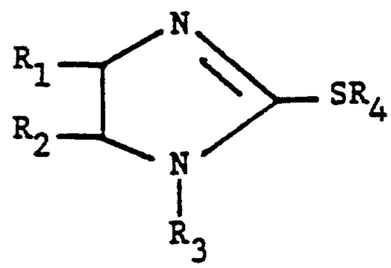

(I) ,

worin $R_1$ und $R_2$ unabhängig voneinander gegebenenfalls substituierte Aryl- oder Heteroarylgruppen bedeuten, $R_3$ Wasserstoff oder Niederalkyl darstellt und $R_4$ einen gegebenenfalls substituierten aliphatischen Kohlenwasserstoffrest bedeutet, und deren Salze.

2. Verbindungen gemäss Anspruch 1, worin $R_1$ und $R_2$ unabhängig voneinander gegebenenfalls substituierte Phenyl-, Thienyl- oder Pyridylgruppen bedeuten, $R_3$ Wasserstoff oder Niederalkyl darstellt und $R_4$ einen gegebenenfalls durch Carboxy bzw. Niederalkoxycarbonyl, gegebenenfalls substituiertes Phenyl oder in einer höheren als der α-Stellung durch Hydroxy, Niederalkoxy, Niederalkylthio oder eine gegebenenfalls substituierte Phenoxy- oder Phenylthiogruppe einfach oder durch Halogen ein- oder mehrfach substituierten aliphatischen Kohlenwasserstoffrest mit bis und mit 7 Kohlenstoffatomen bedeutet, wobei Phenyl-, Phenoxy-, Phenylthio- und Pyridylgruppen durch Niederalkyl,

3. Verbindungen gemäss Anspruch 1, worin $R_1$ und $R_2$ unabhängig voneinander gegebenenfalls durch Niederalkyl mit bis zu 4 C-Atomen, Niederalkoxy mit bis zu 4 C-Atomen oder Halogen mit Atomnummer bis und mit 35 substituiertes Phenyl oder unsubstituiertes Pyridyl bedeuten, $R_3$ Wasserstoff bedeutet, und $R_4$ Niederalkyl mit bis zu 4 C-Atomen, oder ω,ω,ω-Trihalogenniederalkyl mit bis zu 4 C-Atomen bedeutet, und ihre Salze.

4. 2-Aethylthio-4,5-diphenyl-imidazolin oder ein Salz davon.

5. 4,5-Bis-(p-methoxyphenyl)-2-äthylthio-imidazolin oder ein Salz davon.

6. 2-Aethylthio-4,5-bis-(p-chlorphenyl)-imidazolin oder ein Salz davon.

7. Eine Verbindung gemäss einem der Ansprüche 1-11 in der <u>cis</u>-Form, gegebenenfalls in Form eines reinen Antipoden.

8. Eine Verbindung gemäss einem der Ansprüche 1-11 in der <u>trans</u>-Form, gegebenenfalls in Form eines reinen Antipoden.

9. Verfahren zur Herstellung von Verbindung der Formel

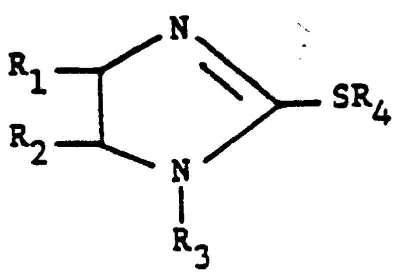

(I) ,

worin $R_1$ und $R_2$ unabhängig voneinander gegebenenfalls substituierte Aryl- oder Heteroarylgruppen bedeuten, $R_3$ Wasserstoff oder Niederalkyl darstellt und $R_4$ einen gegebenenfalls substituierten aliphatischen Kohlenwasserstoffrest bedeutet, und deren Salze, dadurch gekennzeichnet, dass man Verbindungen der Formeln

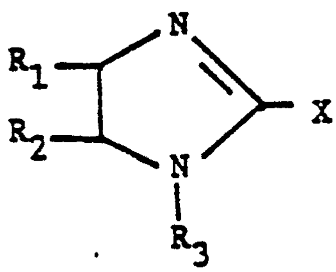 (II) und $Y - R_4$ (III)

worin einer der Reste X und Y gegebenenfalls in Salzform vorliegendes Mercapto und der andere einen gegen veräthertes Mercapto austauschbaren Rest bedeutet, miteinander oder eine Verbindung der Formel II, worin X die Mercaptogruppe darstellt, mit einem gegebenenfalls wie angegeben substituierten Niederalken umsetzt oder eine Verbindung der Formel

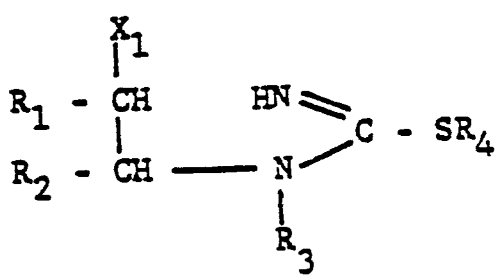 (IV) ;

worin $X_1$ einen gegen Imino austauschbaren Rest bedeutet, oder ein Salz davon cyclisiert oder in einer Verbindung der Formel

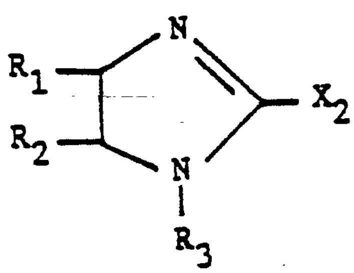 (V) ,

worin $X_2$ einen in einen Rest $R_4S$- überführbaren Rest bedeutet, $X_2$ in den gewünschten Rest $R_4S$- überführt und gewünschtenfalls die erhaltene Verbindung in eine andere Verbindung der Formel I umwandelt, ein gegebenenfalls erhaltenes Stereoisomerengemisch in die Komponenten auftrennt und/oder eine erhaltene freie Verbindung in ein Salz oder ein erhaltenes Salz in die freie Verbindung oder in ein anderes Salz überführt.

10. Pharmazeutische Präparate enthaltend eine Verbindung gemäss einem der Ansprüche 1-9 in freier Form oder in Form eines pharmazeutisch verwendbaren Salzes neben üblichen pharmazeutischen Hilfs- und Trägerstoffen.

Patentansprüche

(für Oesterreich)

1. Verfahren zur Herstellung von Verbindungen der Formel

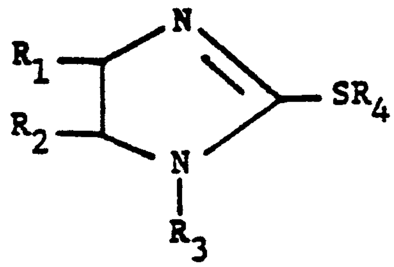

(I) ,

worin $R_1$ und $R_2$ unabhängig voneinander gegebenenfalls substituierte Aryl- oder Heteroarylgruppen bedeuten, $R_3$ Wasserstoff oder Niederalkyl darstellt und $R_4$ einen gegebenenfalls substituierten aliphatischen Kohlenwasserstoffrest bedeutet, und deren Salzen, dadurch gekennzeichnet, dass man Verbindungen der Formeln

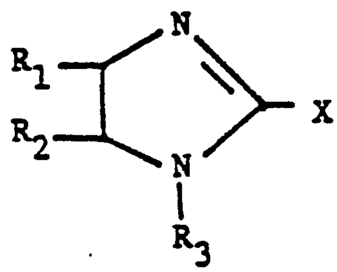

(II) und $Y - R_4$ (III)

worin einer der Reste X und Y gegebenenfalls in Salzform vorliegendes Mercapto und der andere einen gegen veräthertes Mercapto austauschbaren Rest bedeutet, miteinander oder eine Verbindung der Formel II, worin X die Mercaptogruppe darstellt, mit einem gegebenenfalls wie angegeben substituierten Niederalken umsetzt oder eine Verbindung der Formel

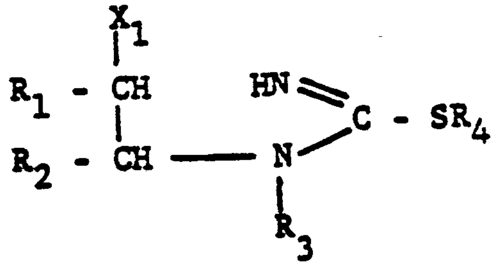

(IV) ,

worin $X_1$ einen gegen Imino austauschbaren Rest bedeutet, oder ein Salz davon cyclisiert oder in einer Verbindung der Formel

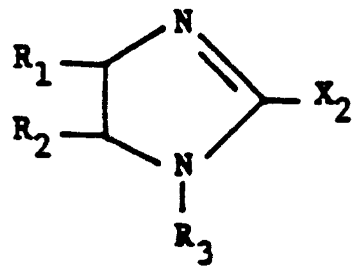

(V) ,

worin $X_2$ einen in einen Rest $R_4S$- überführbaren Rest bedeutet, $X_2$ in den gewünschten Rest $R_4S$- überführt und gewünschtenfalls die erhaltene Verbindung in eine andere Verbindung der Formel I umwandelt, ein gegebenenfalls erhaltenes Stereoisomerengemisch in die Komponenten auftrennt und/oder eine erhaltene freie Verbindung in ein Salz oder ein erhaltenes Salz in die freie Verbindung oder in ein anderes Salz überführt.

2. Verbindungen der Formel I, worin $R_1$ und $R_2$ unabhängig voneinander gegebenenfalls substituiertes Phenyl, Thienyl oder Pyridyl bedeuten, $R_3$ Wasserstoff oder Niederalkyl darstellt und $R_4$ gegebenenfalls durch Phenyl oder in einer höheren als der α-Stellung durch Hydroxy, Niederalkoxy, Niederalkylthio, Phenoxy oder Phenylthio einfach oder durch Halogen bis und mit Atomnummer 35 ein- oder mehrfach substituiertes Niederalkyl oder einen gegebenenfalls durch Phenyl substituierten Niederalkenyl- oder Niederalkinylrest bedeutet, wobei Phenyl-, Phenoxy- und Phenylthiogruppen durch Niederalkyl, Niederalkoxy, Halogen bis und mit Atomnummer 35, Nitro, Amino oder N,N-Di-niederalkylamino und Pyridylgruppen durch Niederalkyl oder Niederalkoxy substituiert sein können, und ihre Salze.

3. Verbindungen gemäss Anspruch 1, worin $R_1$ und $R_2$ unabhängig voneinander gegebenenfalls durch Niederalkyl mit bis zu 4 C-Atomen, Niederalkoxy mit bis zu 4 C-Atomen oder Halogen mit Atomnummer bis und mit 35 substituiertes Phenyl oder unsubstituiertes Pyridyl bedeuten, $R_3$ Wasserstoff bedeutet, und $R_4$ Niederalkyl mit bis zu 4 C-Atomen, oder ω,ω,ω-Trihalogenniederalkyl mit bis zu 4 C-Atomen bedeutet, und ihre Salze.

4. Verfahren gemäss einem der Ansprüche 1-3, dadurch gekennzeichnet, dass man 2-Methylthio-4,5-diphenyl-imidazolin oder ein Salz davon herstellt.

5. Verfahren gemäss einem der Ansprüche 1-3, dadurch gekennzeichnet, dass man 2-Aethylthio-4,5-diphenyl-imidazolin oder ein Salz davon herstellt.

6. Verfahren gemäss einem der Ansprüche 1-3, dadurch gekennzeichnet, dass man 4,5-Bis-(p-methoxyphenyl)-2-äthylthio-imidazolin oder ein Salz davon herstellt.

7. Verfahren gemäss einem der Ansprüche 1-3, dadurch gekennzeichnet, dass man 2-Aethylthio-4,5-bis-(p-chlorphenyl)-imidazolin oder ein Salz davon herstellt.

8. Eine Verbindung gemäss einem der Ansprüche 1-7 in der <u>cis</u>-Form, gegebenenfalls in Form eines reinen Antipoden, herstellt.

9. Eine Verbindung gemäss einem der Ansprüche 1-7 in der <u>trans</u>-Form, gegebenenfalls in Form eines reinen Antipoden, herstellt.

Fassung Österreich

0008073



10. Verfahren dadurch gekennzeichnet, dass man pharmazeutische Präparate enthaltend eine Verbindung gemäss einem der Ansprüche 1-10 in freier Form oder in Form eines pharmazeutisch verwendbaren Salzes neben üblichen pharmazeutischen Hilfs- und Trägerstoffen herstellt.

Europäisches Patentamt

# EUROPÄISCHER RECHERCHENBERICHT

0008073

Nummer der Anmeldung

EP 79 10 2753

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| A | BE - A - 863 758 (DU PONT)(08-08-1978)<br>* Seiten 1-6 *<br>-- | 1,2,10 |
| A | US - A - 2 981 739 (BIMBER)<br>* Spalten 1-3 *<br>-- | 1,2,10 |
| A | DE - A - 2 635 876 (DU PONT)<br>* Seiten 3-8 *<br>---- | 1,2,10 |

KLASSIFIKATION DER ANMELDUNG (Int.Cl. 3)

C 07 D 233/42
401/04
409/04
A 61 K 31/415
31/44

RECHERCHIERTE SACHGEBIETE (Int. Cl. 3)

C 07 D 233/42
401/04
409/04
A 61 K 31/415
31/44

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 12-11-1979 | DE BUYSER |